# EUROPEAN PATENT APPLICATION

(11) **EP 1 258 528 A1**
(43) Date of publication of application: **20.11.2002**
(21) Application number: 01111655.5
(22) Date of filing: 14.05.2001
(51) Int. Cl.: C12N 9/88, C12N 15/60, C12N 15/62, C12N 5/10, C12Q 1/48

(54) **Expression of membrane proteins using an adenylyl cyclase of Mycobacterium tuberculosis**

(71) Applicant: GENOPIA Biomedical GmbH, 66123 Saarbrücken (DE)
(72) Inventor: Schultz, Joachim E., Prof. Dr., 72119 Ammerbuch (DE); Linder, Jürgen, Dr., 72072 Tübingen (DE)
(74) Representative: Patentanwälte Ruff, Wilhelm, Beier, Dauster & Partner

(57) **Abstract**

A method for expression of membrane proteins is provided, wherein at least a portion of a nucleotide sequence coding to the membrane protein is fused to at least a portion of a nucleotide sequence coding for an adenylyl cyclase of *Mycobacterium tuberculosis* or a sequence at least about 70 % identical to said sequence of *Mycobacterium tuberculosis*. In a second step the fused nucleotide sequence is introduced into an organism and/or a living cell. Another aspect of the invention is a method for searching active agents, which interact directly or indirectly with adenylyl cyclases.

## Description

The invention relates to a method for expression of membrane proteins and to a method for searching interacting agents.

Membrane proteins are essential components of biological membranes. For a long time it is known, that these proteins are responsible for an immense number of biological activities. For example the different transport mechanisms found in nature are due to the so-called transport proteins of the membrane. An especially important point is signal transduction, which occurs via the membrane of cells and/or organelles, for example. In general, membrane proteins mediate the most important signal transduction phenomena. Therefore, the investigation of membrane proteins is of special interest since long time ago. This is especially true because the biologically phenomena regarding transport and signal transduction relate to medical and especially to pharmaceutical aspects, for example regarding the delivery of drugs to the point of use. Membrane proteins are very important targets for the development of pharmaceutically active agents. As one example, the membrane anchored adenylyl cyclase plays a key role in the hormone mediated signal transduction wherein second messengers like cyclic adenosine monophosphate (cAMP) are involved. In this signal cascade an hormone-binding receptor activates the so-called G-protein which itself activates the adenylyl cyclase, which is anchored in the plasma membrane. The activated adenylyl cyclase catalyses the formation of cAMP from adenosine triphosphate in the presence of Mg²⁺. cAMP is a prominent second messenger in the cell, which is responsible for the regulation of very many biological processes.

There are several pathological situations in which this adenylyl cyclase signal cascade is disturbed. One example is the disease cholera, which is mediated by the bacterium *Vibrio cholera.* This bacterium produces the so-called cholera toxin, which modulates the above-mentioned G-protein in such a manner that the following component in the signal cascade, the adenylyl cyclase, is constantly activated. This leads to an enormous level of cAMP, which results in the pathological symptoms of the disease cholera. This example shows that there is a special interest in analysing and consequently influencing the activity of membrane proteins, in particular members of the adenylyl cylcase family.

One main problem when investigating membrane proteins is the real impossibility to express such proteins in cellular systems by genetic engineering methods in such a way that the proteins are structural and functional comparable with the native membrane proteins. Consequently the common expression systems are not suited for an investigation of membrane proteins, because the proteins, e.g. enzymes, have lost their native characteristics, e.g. their activity, nothing to say about the structural deficiencies of the recombinant membrane proteins obtained by hitherto methods. Even if membrane proteins may be expressed in low yields by bacterial expression systems they are not inserted into the cell membrane but mostly located to inclusion bodies and intracellular structures, due to improper folding. Correct insertion into cell membranes is crucial for membrane protein function, since many membrane proteins get their native structure only in the environment of the correct phospholipid-bilayer, other associated membrane proteins or glycolipids, only to mention a few examples. Furthermore with the commonly used molecular biological methods it is not possible to obtain sufficient yields of recombinant membrane proteins, which are necessary for crystallographic structural analysis, for example. In this field yields in the range of mg are necessary.

Therefore, the present invention has the object to provide a method for expression of membrane proteins, which are structural and functional comparable with the native proteins. Furthermore the inventive method should be suited for comparatively high yields of recombinant protein, for example with respect to crystallography. This object is solved by a method as described in claim 1. The dependent claims 2 to 9 relate to preferred embodiments of the inventive method. Claims 10 to 13 concern fused nucleotide sequences and fused proteins, respectively. Claim 14 describes a kit and in claims 15 to 21 methods for searching active agents are elucidated. Claim 22 relates to an active agent. The wording of all claims is hereby made to the content of the specification.

The inventive method for expression of membrane proteins is characterized by the fusion of the nucleotide sequence or a portion of this sequence with a nucleotide sequence coding for an adenylyl cyclase of *Mycobacterium tuberculosis* or a portion of this sequence or a sequence, which is at least about 70 % identical so such sequences. In a second step the fused nucleotide sequence is introduced into an organism and/or a living cell, wherein the introduced sequence is expressed by common procedures known by an expert in the art.

According to the present invention the fusion of the sequence for the adenylyl cylclase with any membrane protein leads to the expression of structural and functional correct recombinant proteins, in particular in high-yield-expression systems as *Escherichia coli*. By the inventive method the recombinant membrane proteins'are targeted to the membrane displaying their characteristics like enzymatic activity comparable with the native protein. The term "protein" comprises proteins as well as peptides.

The invention comprises the use of the diverse open reading frames of *Mycobacterium tuberculosis, which* relate to adenylyl cyclase activity in the inventive manner. The diverse sequences are already described in the complete genomic analysis of *Mycobacterium tuberculosis* (Cole et al., 1998, Nature, **318,** 618-624). Especially preferred are the genes Rv2435c and Rv1625c. In an especially preferred embodiment of the invention the sequence Rv1625c (SEQ ID NO 1) is used.

According to the invention the complete sequence as outlined in SEQ ID NO 1 or in SEQ ID NO 2 is used in order to form a fusion product with the sequence coding for any membrane protein of interest. In an especially preferred embodiment of the invention only a portion or several portions of said sequence are used. Especially preferred are portions of the sequence, which code for one ore more parts of the N-terminal domain of the adenylyl cyclase. The N-terminal domain of the adenylyl cyclase comprises six transmembrane spans, which are especially suited in order to target the membrane protein of interest to the membrane in the expression system. According to the invention sequences are used which code for one or more of the transmembrane spans or parts thereof. Furthermore it is preferred to use repeats of portions of the sequences, especially of portions of the sequence coding for the N-terminal domain of the adenylyl cylase.

According to the inventive method it is possible to express any membrane protein wherein the recombinant protein maintains essentially the structural and functional characteristics of the native membrane protein. The membrane protein could be of prokaryotic or eukaryotic origin. In a preferred embodiment of the invention the membrane proteins are enzymes. In an especially preferred embodiment of the invention the membrane proteins are adenylyl cyclases, especially mammalian adenylyl cyclases. Adenylyl cyclases play a crucial role in signal transduction as outlined above. Therefore, there is a special interest in the investigation of these components of signal pathways, especially with respect to the development of pharmaceutically active drugs.

The fusion of the sequence coding for the membrane protein of interest and the nucleotide sequence of *Mycobacterium tuberculosis* or portions of this sequence is performed by known molecular biological methods. The transfer of the sequence is performed by common methods known to an expert in the art. Advantageously, the introduction of the fused nucleotide sequence is performed by the use of an appropriate vector. In general, all common vectors are suited, for example DNA vectors, viral and retroviral vectors. Furthermore the invention comprises transfer methods like liposome mediated gene transfer or electroporation, for example.

In a preferred embodiment of the inventive method the organism, in which the fused nucleotide sequence is introduced and afterwards expressed, is a prokaryotic organism, especially a bacterium. In an especially preferred embodiment the organism is *Escherichia coli. Escherichia coli* is a well-known bacterium widely used in laboratory practice. Bacteria like *Escherichia coli* are easy to cultivate in large amounts. It is therefore especially suited in the inventive manner, for example in order to obtain high yields of recombinant protein. Nevertheless *Escherichia coli* is only one example of suited organisms.

In another preferred embodiment of the invention the organism, in which the fused nucleotide sequence is introduced and afterwards expressed, is a eukaryotic organism or a eukaryotic cell. In an especially preferred embodiment the eukaryotic cell is a mammalian cell. Advantageously cell cultures are used in the inventive manner. Nevertheless all cells and organisms of eukaryotic origin as well as other organisms like fungi are suited. Especially preferred is the use of *Trypanosoma leishmanii*.

One main advantage of the inventive method is, that the recombinant membrane protein can be effectively expressed in prokaryotic as well as in eukaryotic systems. This is independent of the origin of the membrane protein of interest. This is an especially surprising feature of the invention, because up to now, it was very difficult to express e.g. a eukaryotic protein in a prokaryotic system like *Escherichia coli* with sufficient yields in protein activity. As outlined above membrane proteins are particularly difficult to handle. It was not possible to express for example a prokaryotic membrane protein in an active form in another prokaryotic organism, nothing to say of the expression of an active form of a eukaryotic membrane protein in a prokaryotic organism. Now it is possible by the use of the inventive method to provide a structural and functional active membrane protein of prokaryotic origin in a eukaryotic organism and vice versa (so-called heterologous expression).

Furthermore the invention comprises fused nucleotide sequences. Theses sequences comprise a) at least a portion of a nucleotide sequence coding for an adenylyl cyclase of *Mycobacterium tuberculosis* and/or at least a portion of sequence, which is at least about 70 % identical to such sequences and b) at least a portion of a nucleotide sequence coding for any membrane protein. Preferably the sequence coding for the adenylyl cyclase of *Mycobacterium tuberculosis* is Rv1625c and/or Rv2435c. Nevertheless other sequences as outlined above are also possible. In a preferred embodiment the membrane protein is an enzyme. Preferably the fused sequence comprises further components. In an especially preferred embodiment the inventive fused sequences are integrated in an appropriate vector, for example a DNA vector, which is suited for an introduction of the sequence into an appropriate host organism.

Additionally the invention comprises fused proteins. Theses proteins comprise a) at least a portion of a an adenylyl cyclase of *Mycobacterium tuberculosis* and/or at least a portion of an amino acid sequence at least about 70 % identical to an adenylyl cylclase of *Mycobacterium tuberculosis* and b) at least a portion of a any membrane protein. In a preferred embodiment the membrane protein is an enzyme. The fused protein could be a protein expressed in an organism. In a preferred embodiment the fused protein is purified at least partly. Preferably the fused protein is suited for investigations of crystallographic structure.

Another aspect of the invention is a kit for expression of membrane proteins. This kit comprises at least one or more portions of the nucleotide sequence Rv1625c (SEQ ID NO 1) and/or Rv2435c (SEQ ID NO 2) of *Mycobacterium tuberculosis.* Regarding further details of said nucleotide sequence reference is made to the above description. Preferably said sequence is integrated in an appropriate vector. In another embodiment of the kit it comprises further components for performing an expression of membrane proteins. These components are e.g. means for the transfer of the nucleotide sequence, appropriate host cells, buffers and/or media.

Furthermore the present invention comprises a method for searching active agents, which interact directly or indirectly with adenylyl cyclases. According to this inventive method an adenylyl cyclase of *Mycobacterium tuberculosis* is expressed in an organism and/or a living cell. Preferably the expression of said enzyme is obtained by introduction of an appropriate nucleotide sequence into a host organism. Further details of this procedure can be taken form the examples. In a second step of the inventive method a potential active agent is brought into contact with the adenylyl cyclase and finally the influence of the active agent on the adenylyl cylclase is analysed.

As outlined above adenylyl cyclases play a crucial role in signal transduction. Therefore there is a special interest in investigating these membrane-anchored enzymes and to find substances, which are able to influence these important proteins. Up to now it was not possible to express these enzymes in a functional manner in recombinant cellular systems. Surprisingly by the use of the inventive method, wherein the adenylyl cylclase of *Mycobacterium tuberculosis* is expressed, a system with recombinant enzyme is provided, wherein the enzymes maintains essentially its native characteristics. This system is especially suited firstly to investigate adenylyl cyclases in general and secondly to find substances, which influence these enzymes. Such influencing substances, so-called active agents, are e.g. useful for the development of pharmaceutically compositions for the treatment of diseases, which are connected, with a disturbance of the signal cascade involving adenylyl cyclases. One example of such diseases is possibly tuberculosis.

The different nucleotide sequences of *Mycobacterium tuberculosis* coding for adenylyl cyclases are suited for this aspect of the invention. In an especially preferred embodiment of this aspect of the invention the nucleotide sequence coding for the adenylyl cylcase is the gene Rv1625c and/or Rv2435c or portions thereof. In general, all common methods for introducing nucleotide sequences into host cells are possible. In an especially preferred embodiment the nucleotide sequence is introduced into the host organism by the help of an appropriate vector, e.g. a DNA vector or retroviral vector. The host organism can be a prokaryotic organism like *Escherichia coli* for example, or a eukaryotic cell or organism. Advantageously the host organism is a mammalian organism or a mammalian cell. Preferably the host cells are cultivable cells, as often used in laboratory practice.

In the inventive method for searching active agents generally any substance is testable with respect to its influence on adenylyl cyclases. For example whole libraries of natural and/or artificial substances can be analysed in a screening based on the inventive method. In an especially preferred embodiment the potential active agents are selected by the way of computer aided modelling. In a preferred embodiment this method is automated in a way known to experts in the art in order to enable a high through-put-screening.

The influence of the potential active agent on the adenylyl cylase is analysable by different methods. One possibility is to analyse a direct interaction of the membrane anchored enzyme and the potential agent, for example by detection of complex formation. In another especially preferred embodiment the influence is analysed by an altered enzyme activity of the adenylyl cyclase. In this connection the enzyme activity is preferably measured before and after adding the potential agent. Details of the measurement of enzyme activity are described in the examples.

Finally the invention comprises active agents, which interact directly or indirectly with adenylyl cyclases. These active agents are obtained by a method as described above.

The described features of the invention and further features result in greater detail from the following description of examples in combination with the figures and the sub claims. The different features could be realized alone or in combination with each other.

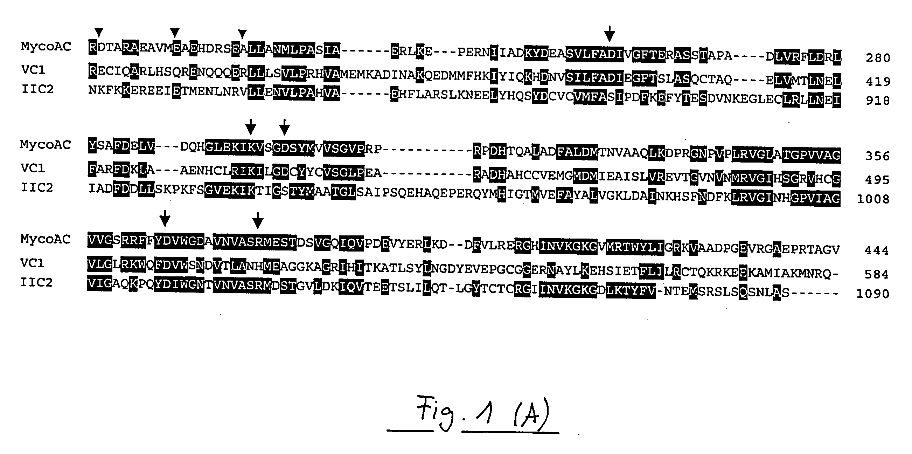

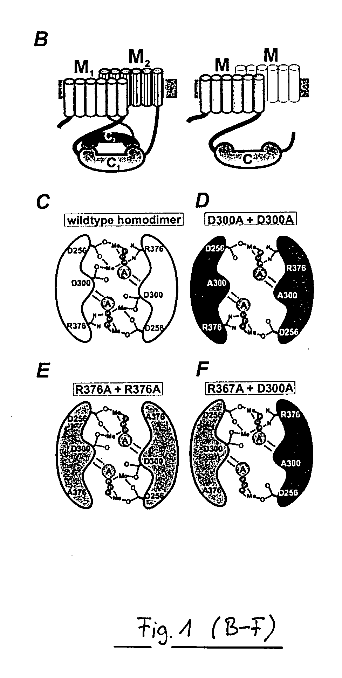

In the figures it is shown
- Fig. 1: (**A**) Alignment of the catalytic domains of the mycobacterial adenylyl cyclase with C₁ from canine type V (V C₁) and C₂ from rat type II (II C₂) adenylyl cyclases (shared residues with either mammalian sequence are inverted). The triangles indicate D204, E213, A221 as starting points of the cytosolic constructs. The arrows mark the mutated amino acids (to alanine), which are involved in substrate definition (K296, D365), coordination of metal ions (D256, D300) and transition state stabilization (R376). In the mammalian domains the equivalents of D256 and D300 are contributed by C₁ whereas D365 and R376 are contributed by C₂. (**B**) Predicted topology of the pseudoheterodimeric mammalian adenylyl cyclases (left) and the monomeric mycobacterial AC. M designates a membrane cassette of six transmembrane spans. In the mycobacterial enzyme, the homodimerization is intimated by a sketchy second M domain. (**C**) Symbolized homodimeric catalytic center of the mycobacterial adenylyl cyclase capable to form two catalytic sites. D256, D300 and R376 are outlined, binding of the adenine ring A is indicated by dotted lines. (**D**) Proposed homodimeric structure of the D300A and (**E**) R376A mutants. (**F**) Symbolized heterodimer with a single catalytic site reconstituted from the D300A and R376A mutant monomers. The same model may be applied to the D256A mutation (not depicted). P = phosphate; Me = divalent metal cation.

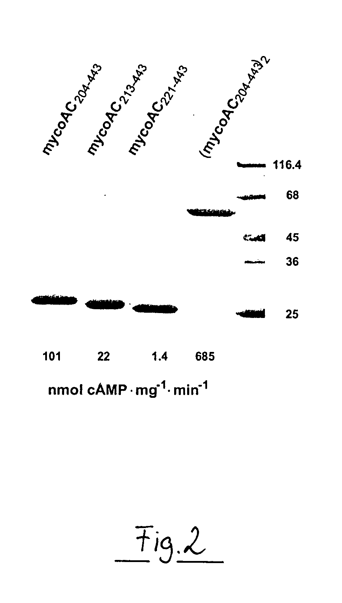

- Fig. 2: Purification and activity of recombinant mycobacterial adenylyl cyclase catalytic domains (SDS-PAGE analysis, Coomassie blue staining). The soluble cytoplasmic domains started at D204 (mycoAC₂₀₄₋₄₄₃), E213 (mycoAC₂₁₃₋₄₄₃) and A221 (mycoAC₂₂₁₋₄₄₃) as indicated in Fig. 1A. The specific activities indicated below each lane were determined with 75 µM ATP at a protein concentration of 2 µg/assay (750 nM). The dimer (mycoAC₂₀₄₋₄₄₃)₂ was assayed at 0.6 µg protein/assay (110 nM).

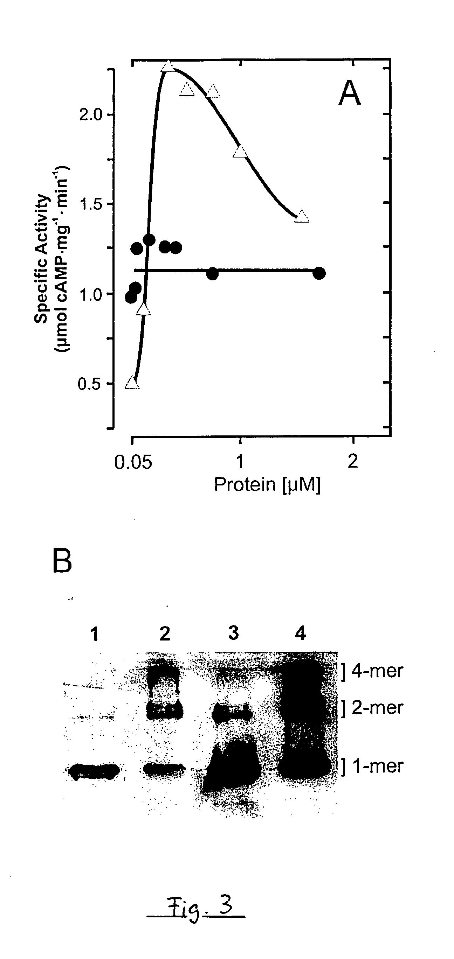

- Fig. 3: (**A**) Protein dependence of mycoAC₂₀₄₋₄₄₃ (Δ) and linked (mycoAC₂₀₄₋ ₄₄₃)₂ (•) activity (850 µM Mn-ATP, 4 min). (**B**) Western blot analysis of mycoAC₂₀₄₋₄₄₃ oligomers cross-linked by 20 mM glutaraldehyde. Lane 1: 300 nM mycoAC₂₀₄₋₄₄₃ (0.24 µg protein), no glutaraldehyde; lane 2: + 20 mM glutaraldehyde; lane 3: 2 µM mycoAC₂₀₄₋₄₄₃ (1.6 µg protein), no glutaraldehyde; lane 4 + 20 mM glutaraldehyde. Densitometric evaluation (lanes 1 to 4): the monomers accounted for 96, 31, 80 and 30, the dimers for 4, 44, 18 and 33 and the tetramers for 0, 25, 2 and 37%, respectively.

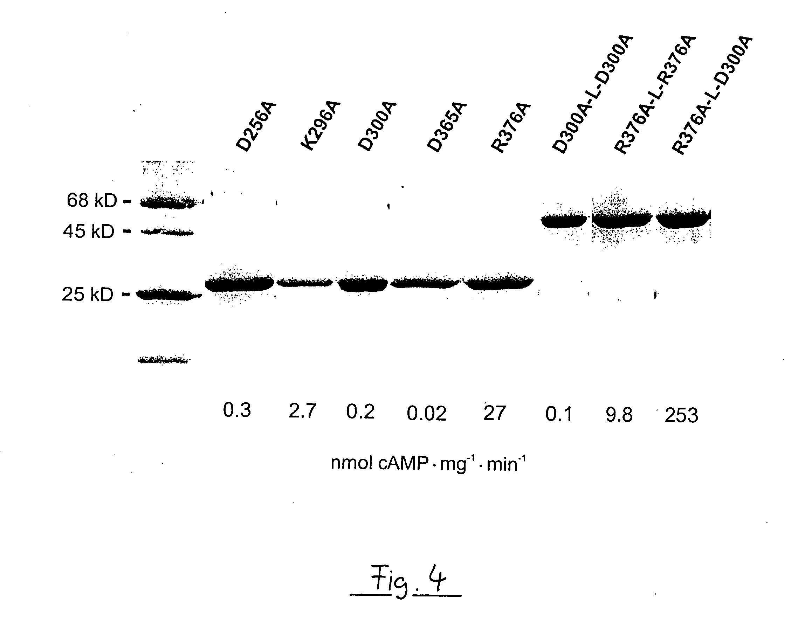

- Fig. 4: SDS-PAGE analysis of purified mycoAC₂₀₄₋₄₄₃ mutant constructs (Coomassie blue staining). Molecular mass standards are at left. The following amounts of protein were applied (left to right): D256A, 4µg; K296A, 1.5 µg; D300A, 4 µg; D365A, 2 µg; R376A, 2.3 µg; D300A-L-D300A, R376A-L-R376A and R376A-L-D300A, 4 µg each. The specific AC activities determined at 75 µM Mn-ATP and 4 µM protein are indicated below each lane (114 nM for R376A-L-D300A).

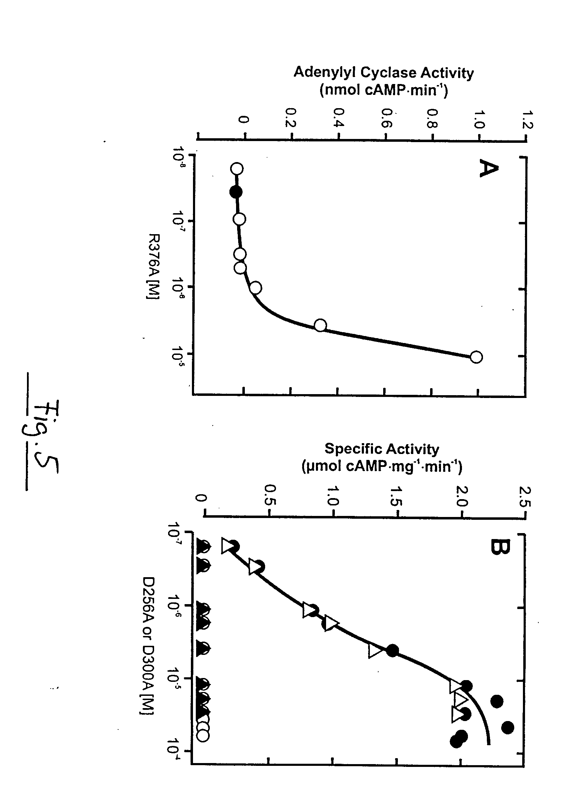

- Fig. 5: Reconstitution of mycobacterial adenylyl cyclase activity from the D256A, D300A and R376A mutants. (**A**) Protein dependency of R376A activity. The protein concentration marked by the filled circle (45 nM) was used in (**B**) for reconstitution with increasing amounts of D256A (Δ) and D300A (•); D256A (▲) and D300A(○) alone. Note the different scales (assays with 850 µM Mn-ATP, 4 min).

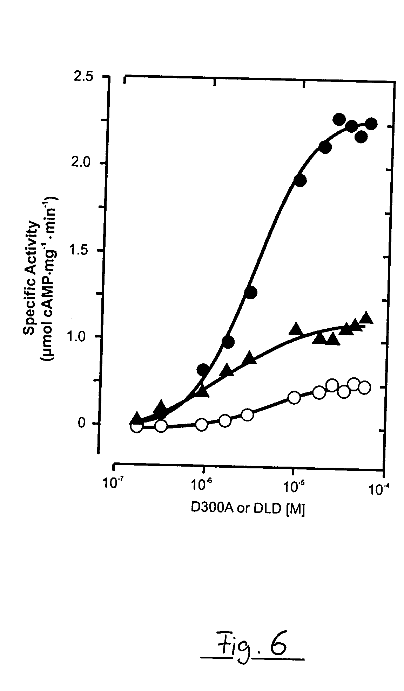

- Fig. 6: Intermolecular formation of adenylyl cyclase catalysts from mutated, inactive monomers and homodimers. (•):45 nM R376A (inactive) titrated with increasing amounts of D300A-L-D300A (DLD). (▲): 36 nM R376A-L-R376A titrated with D300A; (○): 36 nM R376A-L-R376A titrated with D300A-L-D300A. Assays were carried out with 850 µM Mn-ATP as a substrate for 4 min. The specific activity was calculated on the basis of the fixed concentration of the minor constituent.

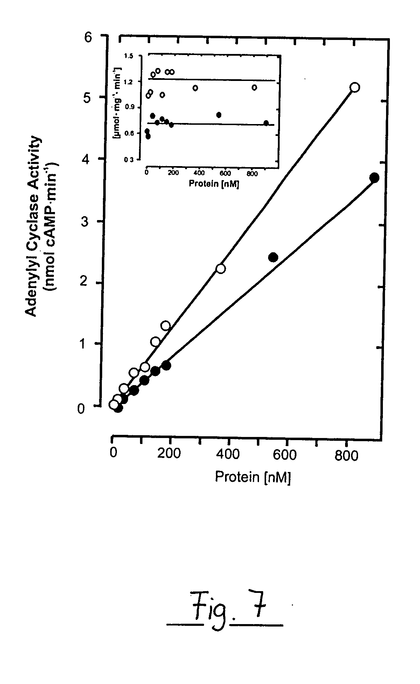

- Fig. 7: One and two catalytic centers formed by (mycoAC₂₀₄₋₄₄₃)₂ (o) and R376A-L-D300A (•), respectively, demonstrated by the linearity of the protein dependences and the unchanging specific activities. Inset: specific activity (same symbols; 850 µM Mn-ATP, 4 min).

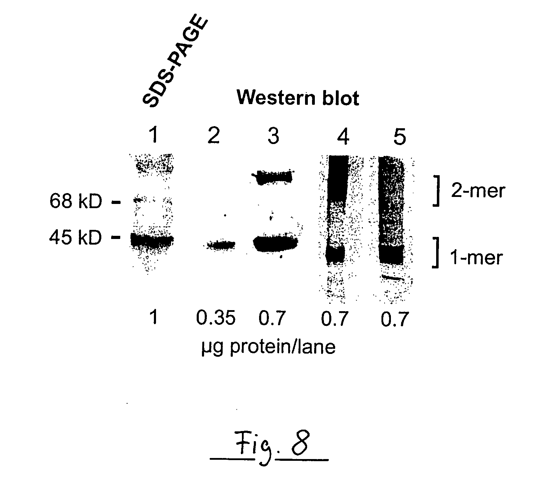

- Fig. 8: Electrophoretic analysis (SDS-PAGE and Western blot) of the mycobacterial adenylyl cyclase holoenzyme. Lane 1: Coomassie blue stained SDS-PAGE of purified enzyme expressed in E. coli; lane 2 and 3: Western blot of purified protein probed with antibodies against the N-terminal His-tag. Note the distinct 92 kDa in lane 3 demonstrating a homodimer. Lane 4: E. coli and (lane 5) HEK293 homogenates expressing the holoenzyme were probed with an antibody directed against mycoAC₂₀₄₋₄₄₃.

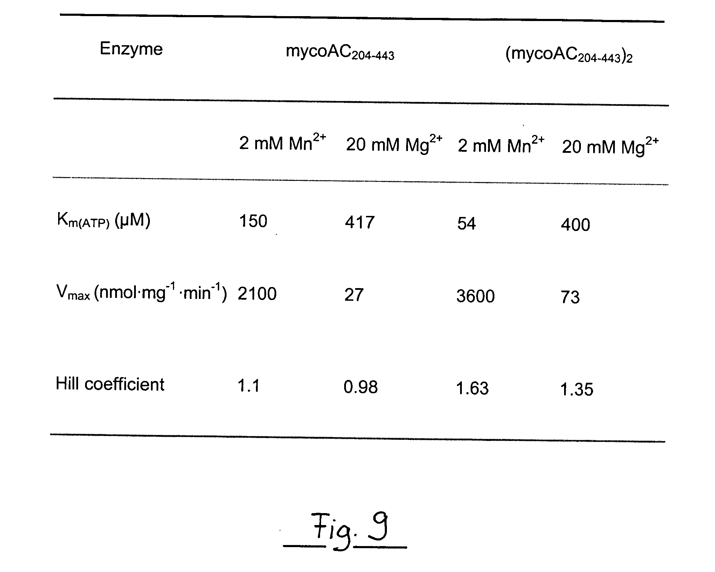

- Fig. 9: Kinetic characterization of the mycoAC monomer and peptide-linked dimer. Assays were conducted at 288 nM (monomer) and 36 nM (dimer) of protein when using Mn²⁺ as a cation (for Mg²⁺ the protein concentrations were 4.4 µM and 196 nM, respectively) (Table I).

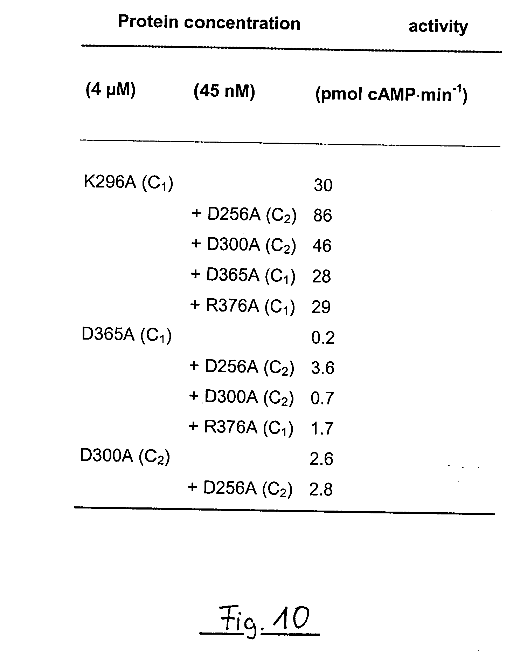

- Fig. 10: AC reconstitution in pairs from point mutated mycoAC monomers. The functional analogies to the C₁ and C₂ catalytic domains of mammalian ACs are bracketed (Table II).

### Examples

### 1. Introduction

In 1998, the complete genome sequence of *Mycobacterium tuberculosis* H37Rv was reported (Cole et al., 1998). Thus, genes of interest are now accessible by PCR using specific primers and genomic DNA as a template. In the *M. tuberculosis* genome 15 open reading frames have been identified which probably code for functional class III adenylyl cyclases (AC) (Cole et al., 1998; McCue et al., 2000). These cyclase isozymes belong to evolutionary segregated branches. Nine are predicted to be similar to ACs found in streptomyces, and four appear to be similar to ACs identified in bacteria such as myxobacterium *Stigmatella aurantiaca* (McCue et al., 2000). Two genes, Rv1625c and Rv2435c, code for proteins which are grouped with the mammalian ACs on a branch separate from other prokaryotic cyclases. The predicted protein of Rv1625c has a molecular mass of 47 kDa. It consists of a large N-terminal membrane domain which is made up of six transmembrane spans, and of a single C-terminal catalytic domain (Tang and Hurley, 1998). As such the predicted protein topology corresponds exactly to one half of a mammalian membrane-bound AC which is a pseudoheterodimer composed of two highly similar domains linked by a peptide chain designated as C_{1b} (Tang and Hurley, 1998; see model in Fig. 1B). The mycobacterial catalytic domain displays considerable sequence identities with those of mammalian ACs (Fig. 1A). This unexpected and so far unique similarity of a bacterial AC to mammalian ACs raises questions about their evolutionary and functional relationship and the pathophysiological role of this *M. tuberculosis* version of a mammalian AC. Additionally, the cloned gene Rv1625c opens novel experimental opportunities because, as shown here, the full length, membrane-bound AC can be actively expressed in mammalian HEK 293 cells as well as in *E. coli*. The expression and characterization of the catalytic center of gene Rv1625c from *M. tuberculosis* is shown and the question of a functional tetrameric structure of ACs as crystallized by Zhang et al. (Zhang et al., 1997) is addressed. All molecular and biochemical properties of the mycobacterial AC monomer Rv1625c indicate that it may constitute a direct progenitor to the mammalian pseudoheterodimeric ACs possibly acquired during the evolution by eukaryotic cells from bacteria via a horizontal gene transfer event (Baltimore, 2001).

### 2. Materials and Methods

### 2.1. Recombinant cDNAs

Genomic DNA from *M. tuberculosis* was a gift of Dr. Boettger, Medical School, Hannover, Germany. The mycoAC gene Rv1625c was amplified by PCR using specific primers and genomic DNA as a template. A BamHI restriction site was added at the 5'-end and a Sacl site at the 3'-end. The DNA was inserted into either the multiple cloning site of pQE30 (Qiagen) with the N-terminal addition of a MRGSH₆GS-peptide sequence or pQE60 which added a C-terminal His-tag. In conjunction with specific primers this clone served as a template to generate by PCR three DNA constructs comprising the catalytic C-terminal starting at D204 (mycoAC₂₀₄₋₄₄₃), E213 (mycoAC₂₁₃₋₄₄₃) or A221 (mycoAC₂₂₁₋₄₄₃). After partial restriction of the products with BamHI and SacI the fragments were ligated into pQE30.

Two catalytic mycoAC₂₀₄₋₄₄₃ segments were linked (C- to N-terminal) via the insertion of a DNA sequence coding for the peptide linker TRAAGGPPAAGGLE using conventional molecular biology techniques. Thus a pQE30 plasmid was generated which coded for the protein MRGSH₆GS-mycoAC₂₀₄₋₄₄₃-TRAAGGPPAAGGLE-mycoAC₂₀₄₋₄₄₃, abbreviated (mycoAC₂₀₄₋₄₄₃)₂.

Single amino acid mutations in the catalytic domain of the mycoAC (D256A, K296A, D300A, D365A and R376A) were introduced by site-directed mutagenesis using respective PCR primers and the DNA coding for mycoAC₂₀₄₋₄₄₃ as a template. Nearby located restriction sites were used as appropriate. The fidelity of all constructs was verified by double-stranded sequencing. The sequences of all primers are available on request.

### 2.2. Expression and purification of bacterially expressed proteins

The constructs in the pQE30 expression plasmid were transformed into *E. coli* BL21(DE3)[pREP4]. For the holoenzyme, the *cya*^{*-*} strain DHP1[pREP4] was used. Cultures were grown in Lennox L broth at 25°C containing 100 mg/liter ampicillin and 25 mg/liter kanamycin and induced with 30 µM isopropyl-β-D-thio-galactopyranoside at an A₆₀₀ of 0.4. Bacteria were harvested after 3 h, washed once with 50 mM Tris-HCI, 1 mM EDTA, pH 8 and stored at -80°C. For purification frozen cells were suspended in 20 ml of cell lysis buffer (50 mM Tris-HCI, 50 mM NaCI, 10 mM β-mercaptoethanol, pH 8) and sonicated (3 x 10 s). Cell debris was removed by centrifugation (48,000 x g, 30 min). To the supernatants 200 µl of nickel-NTA slurry (Qiagen) was added. After gentle rocking for 30 min at 0°C, the resin was poured into a column and washed (2 ml/wash; wash buffer A: 50 mM Tris-HCI, pH 8, 10 mM β-mercaptoethanol, 2 mM MgCl₂, 400 mM NaCI, 5 mM imidazole; wash buffer B: wash buffer A with 15 mM imidazole; wash buffer C: wash buffer A with 10 mM NaCI and 15 mM imidazole). Proteins were eluted with 0.5 to 1 ml of buffer C containing 150 mM imidazole. Because the mycoAC was inhibited by more than 1 mM imidazole, the eluates were dialyzed overnight against 50 mM Tris-HCI, pH 7.5, 2 mM β-mercaptoethanol, 10 mM NaCI and 20% glycerol. The purified proteins could be stored in dialysis buffer at 4°C.

### 2.3. Expression of mycoAC in HEK293 cells

For expression of the mycoAC in HEK293 cells the coding region in pQE60 (Qiagen) containing an RGSH₆ sequence at the carboxy-terminal was excised from pQE60 with Ncol and HindIII, filled in and cloned into the EcoRV site of pIRES1neo (Clontech). HEK293 cells were grown at 37□C in minimum essential medium (Gibco-BRL # 31095-029) containing 10% fetal calf serum and 20 µg/ml gentamicin sulfate. For transfection, the calcium phosphate method was used with 10 µg plasmid DNA/10 cm dish (Kingston et al., 1992). Cells were selected with 800 µg/ml G418 starting 3 days after transfection. After 10 days, the surviving cells were propagated in the presence of 400 µg/ml G418.

For membrane preparation, cells were washed twice with phosphate-buffered saline (PBS; 137 mM NaCI, 2.7 mM KCI, 10 mM Na₂HPO₄, 1.8 mM KH₂PO₄, pH 7.4), suspended and lysed in 250 µl buffer (20% glycerol, 20 mM Tris-HCI, pH 7.5, 1% thioglycerol, 1 mM EDTA and 1 mM benzamidine; two freeze-thaw cycles). The suspension was diluted with 4 volumes of buffer (without glycerol) and sonicated (3 x 10 s, microtip setting 4). After removal of nuclei and debris (6,000 x g) membranes of HEK293 cells were pelleted at 50,000 x g for 30 min.

### 2.4. Adenylyl Cyclase Assay

Adenylyl cyclase activity was measured for 10 min in a final volume of 100 µl (Salomon et al., 1974). The reactions contained 22% glycerol, 50 mM Tris-HCI, pH 7.5, either 2 mM MnCl₂ or 20 mM MgCl₂, the indicated concentrations of [α-³²P]ATP (25 kBq) and 2 mM [2,8-³H]-cAMP (150 Bq). 3 mM creatine phosphate and 1 unit of creatine kinase were used as an ATP-regenerating system when assaying crude extracts. To determine kinetic constants, the concentration of ATP was varied from 10 to 550 µM with a fixed concentration of 2 mM Mn²⁺ or 20 mM Mg²⁺. Assays were preincubated at 30°C for 5 min (37°C with HEK293 membranes) and the reaction was started by the addition of enzyme. Usually less than 10% of the ATP was consumed at the lowest substrate concentrations.

### 2.5. Western blot analysis

Protein was mixed with sample buffer and subjected to SDS-PAGE (15%). Proteins were blotted onto PVDF membranes and sequentially probed with a commercial anti-RGS-His₄ antibody (Qiagen) and with a 1:5000 dilution of a peroxidase-conjugated goat-anti-mouse IgG secondary antibody (Dianova). Peroxidase detection was carried out with the ECL-Plus kit (Amersham-Pharmacia). An affinity-purified, highly specific antibody against mycoAC₂₀₄₋₄₄₃ raised in rabbits was employed to detect AC expression in homogenates from *E. coli* and HEK293 cells.

### 2.6. Cross-linking with glutaraldehyde

The cross-linking of purified mycoAC₂₀₄₋₄₄₃ was carried out in 30 µl at 22°C in 50 mM Na₂HPO₄, 10 mM NaCI, 2 mM MgCl₂, ± 850 µM ATP, 20 mM glutaraldehyde and 20% glycerol, pH 7.4. After 60 min the reaction was quenched by with 5 µl of 1M Tris-HCI, pH 7.5. After addition of 5µl sample buffer, proteins were resolved by 15% SDS-PAGE and detected in a Western blot using the anti-RGS-His₄ antibody.

### 3. Results

The gene Rv1625c from *Mycobacterium tuberculosis* codes for a membrane-anchored adenylyl cyclase corresponding to exactly one half of a mammalian adenylyl cyclase. An engineered, soluble form of Rv1625c was expressed in *E. coli*. It formed a homodimeric cyclase with two catalytic centers. Amino acid mutations predicted to affect catalysis resulted in inactive monomers. A single catalytic center with wild-type activity could be reconstituted from mutated monomers in stringent analogy to the mammalian heterodimeric cyclase structure. The proposed existence of supramolecular adenylyl cyclase complexes was established by reconstitution from peptide-linked, mutation-inactivated homodimers resulting in pseudo-trimeric and - tetrameric complexes. The mycobacterial holoenzyme was successfully expressed in *E. coli* and mammalian HEK293 cells, i.e. its membrane-targeting sequence was compatible with the bacterial and the eukaryotic machinery for processing and membrane insertion. The membrane-anchored mycobacterial cyclase expressed in *E. coli* was purified to homogeneity as a first step toward the complete structural elucidation of this important protein. As the closest progenitor of the mammalian adenylyl cyclase family to date the mycobacterial cyclase probably was spread by horizontal gene transfer.

### 3.1. Sequence analysis of the Rv1625 adenylyl cyclase

The *M. tuberculosis* gene Rv1625c (Genbank accession number AF017731) codes for an AC (mycoAC) with six putative transmembrane-helices as a membrane anchor and a single catalytic domain (M and C; Fig. 1B; Tang and Hurley, 1998). In contrast, the mammalian membrane-bound ACs consist of two different cytoplasmic catalytic domains (C_{1a,b} and C₂) each following a transmembrane segment with six α-helices (M₁ and M₂; Fig. 1B, (Krupinski et al., 1989; Sunahara et al., 1996; Tang and Hurley, 1998). Thus, mammalian ACs are pseudoheterodimers. An alignment of the single mycobacterial catalytic loop with the mammalian C₁ₐ or C₂ catalytic segments revealed extensive similarities (Fig. 1A). The identity to C₁ₐ from canine AC type V was 30 % (73 of 241 amino acids), with conservative replacements the similarity was 40 %. Similar values were obtained in a comparison with C₂ from rat AC type II, 31 % identity (75 of 241) and 45 % similarity (rat type II and canine type V ACs were chosen because in the available x-ray structures these domains were used (Tesmer et al., 1997; 1999; Zhang et al., 1997)). The single catalytic domain of the mycoAC contains each of the amino acids in register which have been identified crystallographically as participating in catalysis, i.e. in the definition of substrate specificity (K296 and D365), in the coordination of two metal ions (D256, D300) and in transition state stabilization (R376). In mammalian ACs those amino acids are contributed either from the C₁ domain (D396 and D440 in canine AC type V for metal coordination) or from the C₂ segment (K938, D1018 for binding to N1 and N6 of the adenine moiety and R1029 to stabilize the transition state in rat AC type II). The gaps of 4, 3 and 11 amino acids in the alignment with the C₂ domain possibly indicate a closer kinship of the mycobacterial catalytic loop to the mammalian C₁ₐ units. Notably, the 11 amino acid gap excludes the QEHA motif which proposedly binds the β,γ subunits of G-proteins (Chen et al., 1995). In the canonical mammalian ACs a dimerization of the cyclase homology domains is required to form the catalytic pocket at the domain interface. Thus the mycobacterial monomer probably functions as a C₁ as well as a C₂ catalytic domain in a homodimer which may form two competent catalytic sites (symbolized in Fig. 1C).

### 3.2. Characterization of a soluble mycobacterial AC homodimer

Three cytosolic segments of different lengths to generate a soluble, enzymatically active catalytic domain from the mycoAC (Tang and Gilman, 1995; Whisnant et al., 1996; Yan et al., 1996) were constructed. The N-terminally His-tagged constructs which started at D204, i.e. at the calculated exit of the last transmembrane span, at E213 and at A221 (marked in Fig. 1A), were expressed in *E. coli* and purified to homogeneity by Ni-NTA chromatography (Fig. 2). At 750 nM of protein, the AC activities were 101, 22 and 1.4 nmol cAMP/mg·min⁻¹ (100, 22 and 1.4%), respectively, i.e. the activities declined upon shortening of the N-terminal ahead of F254, the presumed beginning of the catalytic core. Therefore, mycoAC₂₀₄₋₄₄₃ was used for further investigations.

The specific activity of mycoAC₂₀₄₋₄₄₃ was dependent on the protein concentration. From 50 to 300 nM it increased five-fold suggesting the formation of a homodimer (Fig. 3A). Further increments in the protein concentration resulted in a decrease of the specific activity (Fig. 3A). The dimerization was substantiated with a construct in which two monomers were joined by a flexible tetradekapeptide linker, (mycoAC₂₀₄₋₄₄₃)₂. The dimer was expressed in *E. coli* and purified by Ni-NTA chromatography (Fig. 2). The specific activity of (mycoAC₂₀₄₋₄₄₃)₂ was independent of the protein concentration (Fig. 3A). It was reasoned that the decreasing activity of the monomer at protein concentrations exceeding 300 nM might have been caused by the formation of less productive multimers. Therefore, mycoAC₂₀₄₋₄₄₃ at 0.3 and 2 µM concentrations was treated with 20 mM of the crosslinking reagent glutaraldehyde and protein multimerization was analyzed by Western-blotting (Fig. 3B). The extent of the oligomerization which was substrate-independent (not shown), was dependent on the protein concentration, i.e. differences in the oligomeric state may, at least in part, be responsible for the decreasing AC activity at higher protein concentrations.

The kinetic properties of mycoAC₂₀₄₋₄₄₃ and (mycoAC₂₀₄₋₄₄₃)₂ were analyzed using Mn²⁺ or Mg²⁺ (Fig. 9). The Kₘ values for ATP were considerably lower with Mn²⁺ compared to Mg²⁺. Similarly, the Vₘₐₓ values were highest with Mn-ATP as a substrate with the linked dimer showing a considerably higher value (Fig. 9). The pH optimum (7.2; tested range pH 4-9.5), the temperature optimum (37°C) and the activation energy derived from a linear Arrhenius plot (67.5 kJ/mol; tested range 0-60°C) were similar to the constants reported for the mammalian AC catalysts. The Hill coefficients indicated a slight cooperativity for the linked construct. Mn-GTP was not accepted as a substrate. Not surprisingly, 100 µM forskolin did not activate the mycoAC₂₀₃₋₄₄₃. Forskolin efficiently activates most membrane-bound mammalian ACs by binding to a pseudosymmetric substrate-binding site which in the mycoAC probably is part of a second catalytic site (Tesmer et al., 1997; Zhang et al., 1997).

### 3.3. Generation of an active heterodimeric from a homodimeric AC

The crystal structure of the mammalian AC heterodimer predicts which amino acids of the mycoAC ought to be involved in catalysis (Liu et al., 1997; Sunahara et al., 1998; Tesmer et al., 1997; 1999; Yan et al., 1997; Zhang et al., 1997). In Fig. 1C part of this information has been modeled for a mycobacterial AC homodimer. From such a model testable predictions can be derived. First, the mycoAC should be capable to form two catalytically productive sites. Second, all single mutations of the amino acid residues outlined above should be largely inactivating. Third, any reconstitution of a productive heterodimer from inactive mutated monomers should be restricted to those combinations which enable a mammalian C₁/C₂-like situation and result in the reconstitution of only a single catalytic site (as modeled in Fig. 1F). These possibilities were examined.

In the soluble mycoAC₂₀₄₋₄₄₃ D256, K296, D300, D365 and R376 to alanines were individually mutated. The constructs were expressed in *E. coli* and purified to homogeneity (Fig. 4). The mutant proteins had residual AC activities of less than 5 % of the wildtype monomer when compared under identical assay conditions (Fig. 4, see also Fig. 5). Therefore, they were suitable to investigate by reconstitution experiments the formation of the catalytic fold in analogy to a mammalian C₁/C₂ arrangement. Point mutations which targeted the same functional subdomain and excluded the assembly of a mammalian C₁/C₂-like heterodimer could not effectively reconstitute each other (Fig. 10). Further, mutants which targeted the substrate recognition site, mycoAC₂₀₄₋₄₄₃K296A and mycoAC₂₀₄₋₄₄₃D365A, reconstituted to a limited extent only (Fig. 10).

In contrast AC reconstitution to almost wildtype levels was encountered when mixing mycoAC₂₀₄₋₄₄₃R376A with either mycoAC₂₀₄₋₄₄₃D256A or mycoAC₂₀₄₋₄₄₃D300A because these amino acids contribute to the catalysis from different protein domains very much like in a C₁/C₂ complex. A protein dose-response curve of mycoAC₂₀₄₋₄₄₃R376A showed that it had no detectable AC activity up to 1 µM (Fig. 5A). Above this concentration a very small AC activity was detectable (Fig. 5A). mycoAC₂₀₄₋₄₄₃D256A and mycoAC₂₀₄₋ ₄₄₃D300A were essentially inactive up to 55 µM protein (Fig. 5B). Then 45 nM mycoAC₂₀₄₋₄₄₃R376A, an inactive protein concentration serving as a mammalian C₁-like analog (filled circle in Fig. 5A), was used and increasing amounts of mycoAC₂₀₄₋ ₄₄₃D300A as a presumptive C₂ analog (Fig. 5B) were added. A highly active enzyme was reconstituted in analogy to a C₁/C₂ heterodimer as symbolized in Fig. 1F. Plotting the concentration of mycoAC₂₀₄₋₄₄₃D300A vs. AC activity, an apparent Vₘₐₓ value for the rate limiting amount of mycoAC₂₀₄₋₄₄₃R376A of 2.2 µmol cAMP/mg·min⁻¹ was derived. The apparent K_{d} value of 2 µM for the mycoAC₂₀₄₋₄₄₃D300A/mycoAC₂₀₄₋₄₄₃R376A couple was independent of the ATP concentration in the assay indicating that the association of the monomers was not substrate mediated (not shown). The same type of experiment was carried out with the mycoAC₂₀₄₋₄₄₃D256A mutant serving as a C₂ analog (Fig. 5B). Again, activity with mycoAC₂₀₄₋₄₄₃R376A was reconstituted. The K_{d} was 2 µM and the apparent Vₘₐₓ for the rate limiting amount of mycoAC₂₀₄₋₄₄₃R376A was 2.1 µmol cAMP/mg·min⁻¹. Obviously, the 500- to a 1000-fold excess of mycoAC₂₀₄₋₄₄₃D256A or mycoAC₂₀₄₋₄₄₃D300A over mycoAC₂₀₄₋₄₄₃R376A resulted in the quantitative formation of a productive heterodimeric complex.

### 3.4. Evidence for active pseudo-trimeric and -tetrameric AC species

The membrane-bound mammalian ACs are pseudoheterodimers in which the C₁ and C₂ domains are derived from an intramolecular association. Crystallographically, a tetrameric C₂ structure has been reported (Zhang et al., 1997). The relevance of this tetramer remained unresolved because it has not been possible to experimentally examine the possibility of an intermolecular association, i.e. the formation of catalytic centers by an association of C₁ and C₂ domains originating from different AC proteins. With the available mycoAC mutant proteins such a possibility could be tested. Mutated homodimers linked by a tetradekapeptide chain were generated, i.e. mycoAC₂₀₄₋ ₄₄₃R376A-TRAAGGPPAAGGLE-mycoAC₂₀₄₋₄₄₃R376A (R376A-L-R376A), D300A-L-D300A and R376A-L-D300A. The constructs were expressed in *E. coli* and affinity purified to homogeneity (Fig. 4). D300A-L-D300A was enzymatically dead, R376A-L-R376A had residual activity and R376A-L-D300A was highly active (Fig. 4). R376A-L-D300A supposedly can form a single catalytic center (Fig. 1F). The Kₘ for Mn-ATP was 72 µM, Vₘₐₓ was 1.6 µmol cAMP/mg·min⁻¹ and the Hill coefficient was 0.98. As observed with the (mycoAC₂₀₄₋₄₄₃)₂ homodimer, the protein dependency of the R376A-L-D300A heterodimer was linear and the specific activity was constant over the tested range of protein concentrations from 5 to 800 nM (Fig. 6). The specific activity of R376A-L-D300A was 65% of (mycoAC₂₀₄₋₄₄₃)₂ (1.100 ± 41 vs. 722 ± 25 nmol cAMP/mg-min⁻¹; S.E.M., n=9; Fig. 6). This is convincing evidence that in the linked wildtype homodimer two positive cooperative catalytic centers (Hill coefficient 1.68) are formed and only a single one in the internally complementing mutant protein R376A-L-D300A (Hill coefficient 0.98).

Then it was investigated whether the R376A-L-R376A could be competed by the mycoAC₂₀₄₋₄₄₃D300A monomer or even the D300A-L-D300A dimer with reconstitution of catalytic sites. 36 nM R376A-L-R376A which is inactive, was partially reactivated in a dose-dependent fashion by increasing amounts of mycoAC₂₀₄₋₄₄₃D300A (Fig. 7). Similarly, 36 nM of R376A-L-R376A was partially reconstituted by increasing concentrations of D300A-L-D300A. Thus it was proved that even the mutated dimers interacted productively and formed a competent catalytic complex with a pseudo-trimeric and -tetrameric entity (Fig. 7). Reversely, it was found that 45 nM R376A could be reactivated by increasing amounts of the D300A-L-D300A dimer to form a functional AC catalytic center with a specific activity comparable to wildtype levels (Fig. 7). From the dose response curves apparent K_{d} values in the range of 2 to 4 µM were derived. These K_{d} values were identical with those determined for the mutated monomer pairs (Fig. 5). The differences in the extent of the reconstitution are not apparent at this point. Perhaps unappreciated conformational peculiarities of individual constructs may be involved.

### 3.5. Expression of the membrane-bound mycoAC in E.coli and HEK293 cells

*M. tuberculosis* carries the information for a mammalian type AC which requires membrane targeting. It was e attempted to express the mycoAC holoenzyme, i.e. including its huge membrane anchor, in *E. coli* as well as in mammalian HEK293 cells. So far no bacterial AC has been expressed in a mammalian cell and the bacterial expression and successful membrane targeting of a mammalian membrane-bound AC holoenzyme has not been reported. For expression in *E. coli*, the mycoAC ORF was cloned into pQE30 adding an N-terminal His₆-tag. After transformation and induction a bacterial membrane preparation yielded a high AC activity (19 nmol cAMP/mg·min⁻¹) indicating expression and successful dimerization in the bacterial membrane. This was demonstrated by a Western blot because a substantial amount of the protein remained dimerized during SDS-PAGE (Fig. 8). In the soluble fraction of the bacterial lysate, no AC activity was detectable. The mycoAC was solubilized from the bacterial membrane with 1% polydocanol as a detergent and purified to homogeneity. SDS-PAGE analysis yielded a single, somewhat fuzzy band typical of a membrane protein at 46 kDa and a faint band at 92 kDa representing a dimer (Fig. 8). A band at 92 kDa was consistent with the presence of the homodimer (Fig. 8). The purified holoenzyme had a specific activity of 1.2 µmol cAMP/mg·min⁻¹ using 75 µM Mn-ATP as a substrate.

The mycobacterial protein could also be functionally expressed in stably transfected mammalian HEK293 cells. HEK293 membranes had an activity of 65 nmol cAMP/mg·min⁻¹ compared with 24 pmol cAMP/mg·min⁻¹ in the vector control. The Kₘ for Mn-ATP was 65 µM. The robust expression of mycoAC in HEK293 cells was also detected by a Western blot using an antibody against the cytosolic portion of the protein (Fig. 8). A dimerized protein was not detected in HEK293 cells after SDS-PAGE, possibly due to the different lipid composition of bacterial and mammalian cell membranes. Thus it was established that the monomeric mycobacterial AC Rv1625c not only sequentially and topologically resembled a mammalian, membrane bound, pseudoheterodimeric AC but it could also be successfully expressed, processed and correctly membrane targeted by mammalian cells.

### 4. Discussion

In the mycobacterial genome, 15 putative ACs are detected (Cole et al., 1998; McCue et al., 2000). This unique situation implies that the resulting signal transduction modalities using cAMP as a second messenger are of great importance to the tubercle bacillus. Although it has been reported that in macrophages with ingested mycobacteria cAMP levels are elevated and the phagosome-lysosome fusion is impaired, at this point it is not known in which function any of the mycobacterial cyclases may be required for pathogenesis (Lowrie, 1978). The protein product of Rv1625c was investigated because of its surprising topological identity and sequence similarity to mammalian membrane-bound ACs. The sequence-derived insights are now covered by the biochemical properties of the expressed protein. Dimerization was a prerequisite and sufficient for formation of the catalytic center. The molecular details were in agreement with the crystallographic description of a mammalian heterodimeric catalytic fold as evident from the studies with single amino acid mutants and from the reconstitution experiments. Because the mycoAC most likely is a symmetric homodimer, two identical and equivalent catalytic sites are generated. It is assumed that the measured activity represented that of a basal state. This leaves the question of its regulation in the bacillus to be resolved. One possibility is that the mycoAC uses intermediary proteins like the mammalian ACs. The presence in mycobacteria of proteins resembling the α, β, and γ-subunits of mammalian G-proteins has been suggested (Shankar et al., 1997), yet an evaluation of the mycobacterial genome has not turned-up proteins with evident structural similarities. Therefore, the molecular nature of these proteins remains to be defined. A plausible regulatory input to the membrane-anchored mycoAC may come from lipids of the complex mycobacterial cell envelope because they may affect the monomer/dimer ratio and thus AC activity in the pathogen.

In the mammalian heterodimer, forskolin binds to the catalytic core at the opposite end of the same ventral cleft that contains the active site. Thr410 and Ser942 (rat type II AC numbering) are implicated in forskolin binding (Tesmer et al., 1997). The correspondingly located amino acids in the mycoAC are Asp300 and Asn372 (Fig. 1A). These two amino acids are a prerequisite for the formation of a second substrate-binding site and probably are responsible for the observed lack of a forskolin effect in the mycobacterial AC. Actually, the elimination of the second substrate-binding site in the mammalian ACs and its replacement by a regulatory site renews the question about the presence of an elusive endogenous forskolin-like molecule in eukaryotes.

Several experimental observations have posed the question whether the pseudoheterodimeric mammalian AC might exist as a true dimer. A tetrameric x-ray structure was observed with the cytosolic C₂ segment (Zhang et al., 1997). Further, target-size (Rodbell et al., 1981) and hydrodynamic analysis (Haga et al., 1977; Yeager et al., 1985) showed possible monomer/dimer transitions of the mammalian pseudoheterodimers and immunoprecipitation of recombinant type I AC yielded a functional oligomer (Tang et al., 1995). With the soluble constructs from mammalian ACs it was impossible to address the question of a potential true dimerization of two pseudoheterodimers. A first biochemical test was carried out using linked mutation-inactivated homodimers which were complementary for each other. Biochemically it was demonstrated the formation of catalytic centers within pseudo-trimeric and - tetrameric structures. The findings are consistent with the possibility of tetramers or higher order structures in mammalian ACs. Such processes could add additional levels of AC regulation in eukaryotic cells.

As a highly surprising finding the mycoAC gene could be expressed without further ado equally well in *E. coli* and in mammalian HEK293 cells (Fig. 8). A membrane-targeting signal of this mycobacterial membrane-bound enzyme is compatible with the bacterial as well as the eukaryotic facilities for targeting, transport and insertion into the respective cell membrane.

Finally, bacterial membrane proteins provide attractive systems for structural characterization of homologues of integral membrane proteins present in eukaryotes. This has been convincingly demonstrated with examples such as the bacterial rhodopsin (Deisenhofer et al., 1985), the potassium channel from streptomyces (Chen et al., 1999) and the mechanosensitive channel from Mycobacterium tuberculosis (Chang et al., 1998). The expression in *E. coli* and purification of the mycobacterial homologue of a mammalian AC including its membrane anchor may be seen as the first step toward a structural elucidation of this important protein.

### 5. References

Baltimore, D. (2001) Our genome unveiled. *Nature*, **409,** 814-816.
Chang, G., Spencer, R.H., Lee, A.T., Barclay, M.T. and Rees, D.C. (1998) Structure of the MscL homolog from Mycobacterium tuberculosis: a gated mechanosensitive ion channel. *Science,* **282,** 2220-2226.
Chen, G.Q., Cui, C., Mayer, M.L. and Gouaux, E. (1999) Functional characterization of a potassium-selective prokaryotic glutamate receptor. *Nature,* **402,** 817-821.
Chen, J., DeVivo, M., Dingus, J., Harry, A., Li, J., Sui, J., Carty, D.J., Blank, J.L., Exton, J.H., Stoffel, R.H., Inglese, J., Lefkowitz, R.J., Logothetis, D.E., Hildebrandt, J.D. and lyengar, R. (1995) A region of adenylyl cyclase 2 critical for regulation by G protein beta gamma subunits. *Science,* **268**, 1166-1169.
Cole, S.T., Brosch, R., Parkhill, J., Garnier, T., Churcher, C., Harris, D., Gordon, S.V., Eiglmeier, K., Gas, S., Barry, C.E., Tekaia, F., Badcock, K., Basham, D., Brown, D., Chillingworth, T., Connor, R., Davies, R., Devlin, K., Feltwell, T., Gentles, S., Hamlin, N., Holroyd, S., Hornsby, T., Jagels, K. and Barrell, B.G. (1998) Deciphering the biology of Mycobacterium tuberculosis from the complete genome sequence. *Nature,* **393**, 537-544.
Deisenhofer, J., Epp, O., Miki, K., Huber, R. and H., M. (1985) Structure of the protein subunits in the photosynthetic reaction centre of Rhodopseudomonas viridis at 3 A resolution. *Nature*, **318**, 618-624.
Haga, T., Haga, K. and Gilman, A.G. (1977) Hydrodynamic properties of the beta-adrenergic receptor and adenylate cyclase from wild type and variant S49 lymphoma cells. *J. Biol. Chem.,* **252,** 5776-5782.
Kingston, R.E., Chen, C.A. and Okayama, H. (1992) Calcium phosphate transfection. In Ausubel, F.M., Brant, A., Kingston, R.E., Moore, P., Sudman, J.G., Smith, J.A. and Struhl, K. (eds.), *Short Protocols in Molecular Biology.* John Wiley and Sons, New York, pp. 9.7-9.9.
Krupinski, J., Coussen, F., Bakalyar, H.A., Tang, W.J., Feinstein, P.G., Orth, K., Slaughter, C., Reed, R.R. and Gilman, A.G. (1989) Adenylyl cyclase amino acid sequence: possible channel- or transporter- like structure. *Science,* **244**, 1558-1564.
Liu, Y., Ruoho, A.E., Rao, V.D. and Hurley, J.H. (1997) Catalytic mechanism of the adenylyl and guanylyl cyclases: modeling and mutational analysis. *Proc. Natl. Acad. Sci. U.S.A.*, **94**, 13414-13419.
Lowrie, D.B. (1978) Tubercle bacilli in infected macrophages may inhibit phagosome-lysosome fusion by causing increased cAMP concentrations. In Folco, G. and Paoletti, R. (eds.), *Molecular biology and pharmacology of cyclic nucleotides.* Elsevier, Amsterdam, pp. 311-314.
McCue, L.A., McDonough, K.A. and Lawrence, C.E. (2000) Functional classification of cNMP-binding proteins and nucleotide cyclases with implications. for novel regulatory pathways in Mycobacterium tuberculosis. *Genome Res.,* **10**, 204-219.
Rodbell, M., Lad, P.M., Nielsen, T.B., Cooper, D.M., Schlegel, W., Preston, M.S., Londos, C. and Kempner, E.S. (1981) The structure of adenylate cyclase systems. *Adv. Cyclic Nucleotide Res.,* **14**, 3-14.
Salomon, Y., Londos, C. and Rodbell, M. (1974) A highly sensitive adenylate cyclase assay. *Anal. Biochem*., **58**, 541-548.
Shankar, S., Kapatral, V. and Chakrabarty, A.M. (1997) Mammalian heterotrimeric G-protein-like proteins in mycobacteria: implications for cell signalling and survival in eukaryotic host cells. *Mol. Microbiol*., **26,** 607-618.
Sunahara, R.K., Beuve, A., Tesmer, J.J., Sprang, S.R., Garbers, D.L. and Gilman, A.G. (1998) Exchange of substrate and inhibitor specificities between adenylyl and guanylyl cyclases. *J Biol Chem,* **273,** 16332-8.
Sunahara, R.K., Dessauer, C.W. and Gilman, A.G. (1996) Complexity and diversity of mammalian adenylyl cyclases. *Annu. Rev. Pharmacol. Toxicol.,* **36,** 461-480.
Tang, W.J. and Gilman, A.G. (1995) Construction of a soluble adenylyl cyclase activated by Gsa and forskolin. *Science,* **268**, 1769-1772.
Tang, W.J. and Hurley, J.H. (1998) Catalytic mechanism and regulation of mammalian adenylyl cyclases. *Mol. Pharmacol.,* **54,** 231-240.
Tang, W.J., Stanzel, M. and Gilman, A.G. (1995) Truncation and alanine-scanning mutants of type I adenylyl cyclase. *Biochemistry,* **34**, 14563-14572.
Tesmer, J.J., Sunahara, R.K., Gilman, A.G. and Sprang, S.R. (1997) Crystal structure of the catalytic domains of adenylyl cyclase in a complex with Gsa-GTPgS. *Science,* **278,** 1907-1916.
Whisnant, R.E., Gilman, A.G. and Dessauer, C.W. (1996) Interaction of the two cytosolic domains of mammalian adenylyl cyclase. *Proc. Natl. Acad. Sci. U.S.A.*, **93,** 6621-6625.
Yan, S.Z., Hahn, D., Huang, Z.H. and Tang, W.J. (1996) Two cytoplasmic domains of mammalian adenylyl cyclase form a Gsa- and forskolin-activated enzyme in vitro. *J. Biol. Chem.,* **271,** 10941-10945.
Yan, S.Z., Huang, Z.H., Shaw, R.S. and Tang, W.J. (1997) The conserved asparagine and arginine are essential for catalysis of mammalian adenylyl cyclase. *J. Biol. Chem.,* **272**, 12342-12349.
Yeager, R.E., Heideman, W., Rosenberg, G.B. and Storm, D.R. (1985) Purification of the calmodulin-sensitive adenylate cyclase from bovine cerebral cortex. *Biochemistry,* **24,** 3776-3783.
Zhang, G., Liu, Y., Ruoho, A.E. and Hurley, J.H. (1997) Structure of the adenylyl cyclase catalytic core. *Nature,* **386,** 247-253.

## Claims

1. Method for expression of membrane proteins, **characterized by** the following steps:
- at least a portion of a nucleotide sequence coding for the membrane protein is fused to at least a portion of the nucleotide sequence Rv1625c (SEQ ID NO 1) and/or Rv2435c (SEQ ID NO 2) of *Mycobacterium tuberculosis* coding for an adenylyl cyclase and/or at least a portion of a nucleotide sequence at least about 70 % identical to Rv1625c and/or Rv2435c and
- the fused nucleotide sequence is introduced into an organism and/or a living cell.

2. Method according to claim 1, **characterized in that** said portion of the nucleotide sequence Rv1625c and/or Rv2435c codes for at least a part of the N-terminal domain of the adenylyl cyclase.

3. Method according to claim 1 or claim 2, **characterized in that** said portion of the nucleotide sequence Rv1625 and/or Rv2435c codes at least partly for one or more transmembrane spans of the adenylyl cyclase.

4. Method according to one of the preceding claims, **characterized in that** said membrane proteins are enzymes.

5. Method according to one of the preceding claims, **characterized in that** said membrane proteins are adenylyl cyclases, especially mammalian adenylyl cyclases.

6. Method according to one of the preceding claims, **characterized in that** said introduction of the nucleotide sequence is performed by use of an appropriate vector.

7. Method according to one of the preceding claims, **characterized in that** said organism and/or living cell is a bacterium, especially *Escherichia coli*.

8. Method according to one of claims 1 to 6, **characterized in that** said organism and/or living cell is at least one eukaryotic cell, especially a mammalian cell.

9. Method according to one of the preceding claims, **characterized in that** said expression is a heterologous expression.

10. Fused nucleotide sequence, comprising
- at least a portion of the nucleotide sequence Rv1625c (SEQ ID NO 1) and/or Rv2435c (SEQ ID NO 2) of *Mycobacterium tuberculosis* coding for an adenylyl cyclase and/or at least a portion of a nucleotide sequence at least about 70 % identical to Rv1625c and/or Rv2435c and
- at least a portion of a nucleotide sequence coding for a membrane protein.

11. Fused nucleotide sequence according to claim 10, **characterized in that** said membrane protein is an enzyme.

12. Fused protein, comprising
- at least a portion of an adenylyl cyclase of *Mycobacterium tuberculosis* and/or at least a portion of an amino acid sequence at least about 70 % identical to an adenylyl cyclase of *Mycobacterium tuberculosis* and
- at least a portion of a membrane protein.

13. Fused protein according to claim 12, **characterized in that** said membrane protein is an enzyme.

14. Kit for expression of membrane proteins, comprising
- at least a portion of the nucleotide sequence Rv1625c (SEQ ID NO 1) and/or Rv2435c (SEQ ID NO 2) of *Mycobacterium tuberculosis*.

15. Method for searching active agents which interact directly or indirectly with adenylyl cyclases, **characterized by** the following steps:
- an adenylyl cyclase of *Mycobacterium tuberculosis* is expressed in an organism and/or a living cell,
- a potential active agent is brought in contact with the adenylyl cyclase,
- an influence of the active agent on the adenylyl cyclase is analysed.

16. Method according to claim 15, **characterized in that** the expression of the adenylyl cylase is performed by an introduction of a nucleotide sequence coding for the adenylyl cyclase, especially the sequence Rv1625c (SEQ ID NO 1) and/or the sequence Rv2435c (SEQ ID NO 2), or portions thereof, into an organism.

17. Method according to claim 16, **characterized in that** said introduction of the nucleotide sequence is performed by use of an appropriate vector.

18. Method according to one of claims 15 to 17, **characterized in that** said organism is a bacterium, especially *Escherichia coli*.

19. Method according to one of claims 15 to 17, **characterized in that** said organism and/or living cell is at least one eukaryotic cell, especially a mammalian cell.

20. Method according to one of claims 15 to 19, **characterized in that** said influence is analysed by a direct interaction of the adenylyl cyclase and the potential active agent.

21. Method according to one of claims 15 to 20, **characterized in that** said influence is analysed by an altered enzyme activity of the adenylyl cyclase.

22. Active agent which interacts directly or indirectly with adenylyl cyclases, **characterized in that** it is obtained by a method according to one of claims 15 to 21.


SEQUENCE LISTING

<110> Völkel Dr., Helge

<120> Expression of membrane proteins

<130> P 41 301 EP

<140>
<141>

<160> 2

<170> PatentIn Ver. 2.1

<210> 1
<211> 2806
<212> DNA
<213> Mycobacterium tuberculosis

<400> 1
ctttgggtgt tccgagggtt tccagcacga cttcggccac ccgcttcatg gtggtgcgcc 60
gatccatggc ggcacgttga atccacttga aagcgtccgg ctcggtcatc ccatgtttgg 120
tctgcagcag gccttttgct cgttccacca gcttgcgggt ttccaaccgt tcagatagcg 180
tcgccacctc gccttccaac gcggtgatct ccctgaaccg gctgaccgcc aattcaatcg 240
ctggaatcag gtcgctgatg ctgaaaggct ttaccaggta tgccatcgcc ccggcatcac 300
gcgcacgttc gaccagatca cgctggctga acgcggtcag caccacgatc ggggcaatac 360
gtttgctggc gatttcggat gcggcgtcga tcccgtcccg gcgcggcatc ttcacgtcca 420
tgatcaccag gtcgggcttg tgcagctcgg ccagctcgac ggcttcctgg ccgtcgccgg 480
cctcgccgac aatttcatat ccctcctctc gcaacatctc ggccaggtcc atgcggatga 540
gcgcttcatc ttccgcgatc aagacccgac gtgggacagc ggcatcggcg tcggtggtgg 600
ggccggtcat gacggacatt gtgtcgtgag cgggcgcgat cgggaacctc gggggcggt 660
gagtcgggac gtggctcctc tagagtagga gaccggatca agccctcgta tcccaactgg 720
cagaggaaac ggactcaaaa cccgtccagt gtgggttcga atcccaccga gggcaccaag 780
cgaacgcacc cggaaagcgc cgacagagtc ggtaaacgct aactcaccga ttaggcttta 840
gttcgtggcg gcaagaaaat gcggcgcccc accgatagca gcggatggct cgacacgacg 900
cccagactgt gtcacggccg tgcgcactca ggcccgcgct cccacgcagc actacgccga 960
aagcgtcgcg cggcggcagc gcgtgcttac catcaccgcc tggctcgctg tcgtggtaac 1020
cgggagcttc gcgctaatgc aactagcgac cggggccggg ggctggtaca tcgccctgat 1080
caacgtcttc accgcggtta ctttcgcgat cgtgcccctg ctacatcgct tcgggggatt 1140
ggtggcaccg cttacgttca tcggcaccgc ctatgtggcg atcttcgcca tcggctggga 1200
tgtgggcacc gacgcgggtg cccagttctt cttcttggta gccgccgcgt tggtggtgtt 1260
gctggtcggc atcgaacaca ccgcgttagc ggtgggccta gcggcggtcg ccgccggtct 1320
ggtgattgcc ctggagttcc tggtcccgcc cgatactggg cttcagccgc cctgggccat 1380
gtcggtaagt ttcgtcctca cgaccgtctc cgcctgcggg gtagcggtcg caacggtgtg 1440
gttcgcgctg cgtgatactg cgcgcgcgga ggcggtcatg gaggcggagc acgaccgctc 1500
cgaagcactg ctggccaaca tgttgccggc cagcatcgcc gagcggctta aagaacccga 1560
gcgaaacatc atcgccgata aatacgacga ggcctcggtg ctgttcgccg acatcgtcgg 1620

gttcaccgaa cgcgccagta gcaccgcgcc ggcggacctg gtccggttcc tggaccgcct 1680
ctacagcgct ttcgacgagc tggtcgacca gcacgggctg gagaaaatca aggtcagcgg 1740
ggactcctac atggttgtca gcggcgttcc gcggccccgg cctgaccata cgcaagcact 1800
ggcggacttt gcgctcgaca tgaccaatgt cgcagcgcaa ttgaaggatc cacgcggcaa 1860
cccggtgccg ctgcgggtgg gcctggccac cggcccggtg gtggcgggtg tggtgggttc 1920
tcgacggttc ttctacgacg tgtggggcga cgcggtcaat gtcgcgtcgc ggatggaatc 1980
caccgattcg gtggggcaaa ttcaggtgcc agacgaggtt tacgagcgtc tcaaggacga 2040
cttcgtgttg cgcgagcgcg gccacataaa cgtcaagggg aaaggcgtaa tgcgcacgtg 2100
gtacctcatc ggccgcaagg tagccgcaga tcccggcgag gtgcgcgggg ccgaaccccg 2160
cacggcaggg gtctgaccgc caagcgctat cccggggccg ctacatgtgt cacactgcgc 2220
ccatggaacc aagccctgtc gtgagccctc tcccaaggtt gttgccgcat ctctggaagt 2280
ccactctggc atcgggaatt ctgtcgctga tcctgggcgt cctggtgctg gcctggccag 2340
gaatatccat tctcgttgct gccatggcgt tcggtgtcta cctcctgatt accggtgtcg 2400
ctcaggttgc gttcgcgttc agcctgcatg tttcggcggg cggccggatc ctgttgttca 2460
tcagcggtgc cgcctcactc atcctggctg tgctggcctt tcgccatttc ggtgacgcgg 2520
ttctgttgct ggccatctgg atcggcatcg ggttcatctt ccgcggcgtc gccaccacgg 2580
tttcggcgat tagtgatccg atgctgccgg gacggggatg gtccatcttc gtcggcgtga 2640
tcagcctcat cgccggcatt gtggtgatgg catcgccgtt cgagtccatc tggatactgg 2700
cgttggtcgt cggcatctgg ctcgtggtga tcggcacatg cgagatcgcg tcgtcgttcg 2760
ccatccgcaa ggcttcgcag acgctcggct gaccgcagaa tcgcgg 2806

<210> 2
<211> 2193
<212> DNA
<213> Mycobacterium tuberculosis

<400> 2
ttgacgtcgg gtgaggcact ggactcggta gccgagagtg aatccacccc ggctaagaag 60
cgccataaga atgtgctccg gcgtcggccg cgtttccggg ccagcatcca gtccaagctc 120
atggtgctgc tgctgttgac gagtatcgtg tccgtcgcgg cgattgcggc cattgtctat 180
caatctggtc gcacttcgct aagagcagcc gcctacgagc ggttgaccca gttgcgcgag 240
tcgcagaagc gggcagttga gacactattt tctgacctga cgaattcgct ggtcatttac 300
gaacgtggac tcacggttgt cgatgccgtc gtgcggttca cggccggctt tgaccagctg 360
gctgacgcca cgatcagccc cgcccaacaa caggcgatcg tcaactacta caacaacgaa 420
ttcatcacac ccgtcgaacg cacgaccggc gataaactcg acatcaccgc gctgctgccg 480
acttctccgg cccaaaggta tcttcaggcg tactacactg caccattcac gtcggaccaa 540
gatgcgatgc ggctggacga tgccggcgac ggcagtgcat ggtcggccgc caacgcgcaa 600
ttcaacagct atttccggga aatcgtcacc cggttcgatt acgacgacgc ggtattgctg 660
gacacccggg gcaatatcgt ctataccctg agcaaggacc ccgacctcgg taccaacatt 720
ctgaccgggc cgtatcgcga atccaatctg cgtgacgcct accttaaagc gttgggcgcc 780
aacgccgtcg actttacctg gattaccgac ttcaagccgt atcagcctca actcggcgtg 840
ccgaccgcgt ggttggtggc accggtcgaa gcgggcggca aaactcaggg cgttttggcg 900
ctgccgttgc cgatcgacaa gatcaataag atcatgaccg ccgacaggca atggcaagcg 960
gctggcatgg gtagtgggac ggaaacctat ctcgccggtc cggacagtct gatgcggtcc 1020
gattctcggc tcttcctgca agacccggag gaataccgga aacaggttgt ggcagcaggc 1080
acgtcacttg atgtggtcaa cagagcgatc cagttcggtg ggacgacgct gctgcagcct 1140
gttgcgaccg aaggactgcg cgccgcccaa cgcggacaga ccggaaccgt cacctccacc 1200

gactacacgg gtagcaggga actggaggcc tacgcgccgc tgaatgtgcc ggactccgat 1260
ctgcactggt cgatcctggc aacgcggaac gattctgagg cgttcgcggc cgtcgcgtcg 1320
ttcagcaggg cgcttgtgct ggttacagtt ggcatcattg tcgtcatctg tgtggcgtcg 1380
atgctgatcg cccatgcgat ggtgcggcca atccggcggc tcgaggttgg cacccagaag 1440
atcagcgcag gcgactacga agtcaacatt ccggtaaagt cacgcgacga aatcggtgat 1500
cttacagccg ctttcaacga gatgagtcgg aatctgcaaa ccaaagagga gctgctcaac 1560
gagcaacgca aggaaaacga ccggttattg ctatcgatga tgcccgagcc agttgtcgag 1620
cggtaccgcc ttggggagca gaccattgcg caggagcacc aagatgtcac cgtcctgttt 1680
gccgacatcc tgggtgtcga cgagatttcc agcggcctgt cgggtaacga actggtcaaa 1740
attgtcgacg agctggtccg ccagttcgat tcggccgccg aacaccttgg tgtcgaacgc 1800
attcgcacgc tgcacaatgg ctatctcgcc ggttgcgggg taaccacgcc acggctggac 1860
aatattcccc gaaccgtcga cttcgcccta gagatgcggc gcatcgtcga tcggttcaat 1920
tgccaaaccg gtaacgatct gcacctgcga gtcggtatca acaccgggga cgtcattagc 1980
gggctggtcg gcagatcaag tgtcgtctac gacatgtggg gcgcggcagt gagtctggcc 2040
taccaaatgc acagcggttc accacagccc ggcatctatg tcacctcgca ggtgtatgag 2100
gcgatgcgag acgtgtggca gttcacggct gcgggcacga tttctgtcgg agggttagaa 2160
gagccgatct accgattgtc ggagcgatca tga 2193